# EUROPEAN PATENT APPLICATION

(11) **EP 4 276 443 A1**
(43) Date of publication of application: **15.11.2023**
(21) Application number: 22172940.3
(22) Date of filing: 12.05.2022
(51) Int. Cl.: G01N 21/17, G01N 21/47, G01N 21/65, G01N 21/21, A61B 5/00, G01B 9/02091, G01N 29/24, G01N 33/543, B82Y 15/00, B82Y 30/00

(54) **MEASUREMENT METHOD AND MEASUREMENT ARRANGEMENT FOR THE REDUCTION OR SUPPRESSION OF UNWANTED BACKGROUND IN IMAGING TECHNIQUES**

(71) Applicant: AIT Austrian Institute of Technology GmbH, 1210 Wien (AT)
(72) Inventor: Schrittwieser, Stefan, 1190 Wien (AT); Hainberger, Rainer, 1100 Wien (AT)
(74) Representative: Wildhack & Jellinek Patentanwälte

(57) **Abstract**

A measurement method and a measuring arrangement for the reduction or suppression of unwanted background in imaging techniques based on a contrast agent employing an optical interrogation that exploits the orientation-dependent optical scattering and/or absorption characteristics of the contrast agent with respect to light, which method comprises the steps of:
- providing a sample (5) to be analyzed in an optical path between a light source (1) and a detector (9),
- providing as contrast agent nano- or microparticles with magnetic and/or electrostatic properties within the sample,
- generating a magnetic and/or electrostatic field of at least one spatial direction,
- generating at least two signals or images, wherein in one signal there is a minimum of the signal of the contrast agent and in the other a maximum of the signal of the contrast agent exploiting the orientation dependent polarization characteristics of the contrast agent,
- subtracting one signal from the at least one other signal to eliminate the background, wherein the nano- or microparticles used as contrast agent have anisotropic geometry and that the nano- or microparticles show geometric uniformity.

## Description

The invention relates to a measurement method for the reduction or suppression of unwanted background in imaging techniques based on a contrast agent employing an optical interrogation that exploits the orientation-dependent optical scattering and/or absorption characteristics of the contrast agent with respect to light, which method comprises the steps of:
- providing a sample to be analyzed in an optical path between a light source and a detector,
- providing as contrast agent nano- or microparticles with magnetic properties within the sample,
- generating a magnetic field of at least one spatial direction,_{[SS1][SS2]}
- generating at least two signals or images, wherein in one signal there is a minimum of the signal of the contrast agent and in the other a maximum of the signal of the contrast agent,
- subtracting one signal from the at least one other signal to eliminate the background. The invention also relates to a measuring arrangement for measurement of optical properties of a sample to be investigated, as well as of analytes contained in the sample, comprising a measuring cell containing the sample, wherein the measuring cell is placed in an optical path between a light source and a detector for the detection of scattered light coming from the light source and interacted with the sample, wherein sample contains a contrast agent, which contrast agent comprises particles that are able to be aligned spatially depending on the presence of a predetermined magnetic or electric field, and wherein at least one electromagnetic device is located adjacent to the measuring cell to be able to act on the contrast agent.

There are a number of optical measurement methods employed for the visualization of material structures and chemical compositions or for the detection of target analytes. Examples for imaging techniques are optical coherence tomography (OCT) and photoacoustic imaging (PAI), while Raman spectroscopy can be employed for the sensitive detection of molecular species. For all mentioned methods, nano- and microparticles with specific optical properties (e.g. plasmon resonance) are used as contrast agents to enhance the signal-to-noise ratio [Ref. 1 to 4]. Despite the use of a contrast agent, a background signal is present due to the material surrounding the particles. This background always smears out the signal of interest. In order to be able to distinguish the signals that are generated by a contrast agent from the background, signals (or images) are compared in practice before and after the addition of the contrast agent [Ref. 5]. On the one hand, this is time-consuming and resource-intensive, and, on the other hand, it is prone to even small changes in the background.

Another method uses magnetic particles as a contrast agent for imaging [Ref. 6 to 10]. Contrary to the method presented here, the signal generation is limited to the movement of the nanoparticles and does not exploit any optical effects of the nanoparticles themselves. The generation of signals is based on causing the surrounding tissue to vibrate, which can then be detected.

It is accordingly an object of the invention to provide a measurement method for the reduction or suppression of unwanted background in imaging techniques, which overcomes the above-mentioned disadvantages of the prior art methods. The method should provide for a simple and affordable solution.

With the foregoing and other objects in view there is provided, in accordance with the invention, a measurement method for the reduction or suppression of unwanted background in imaging techniques wherein the nano- or microparticles used as contrast agent have anisotropic geometry and that a of nano- or microparticles show geometric uniformity.

In an embodiment of the invention cylindrical rod-shaped nano- or microparticles are used.

In a further embodiment of the invention the excitation of the sample an of the nano- or microparticles is conducted with polarized light.

It is also possible to control the polarization in a predefined way.

It is furthermore possible to use non-polarized light for excitation in combination with a polarization filter after the sample.

In an embodiment of the invention the optical scattering is at least partly inelastic.

In a further embodiment of the invention nano- or microparticles show plasmon resonances.

It is also possible to generate an external homogenous low-gradient magnetic field by two permanent magnets located equidistant to the sample or by a pair of coils, preferably arranged in a Helmholtz geometry.

It is furthermore possible to time-modulate the magnetic field.

In an embodiment of the invention an external homogenous low-gradient electric field is generated by two electrodes located equidistant to the sample.

In a further embodiment of the invention the electric field is a time-modulated field.

It is also possible to conduct the signal detection with simultaneous knowledge of the current orientation of the nano- or microparticles.

It is furthermore possible that the detection method is light scattering-based detection schemes.

Alternatively, the detection method is light absorption-based detection schemes.

In an embodiment of the invention the detection method is optical coherence tomography.

In a further embodiment of the invention the detection method is photoacoustic imaging.

It is also possible that the detection method is surface enhanced Raman spectroscopy.

With the foregoing and other objects in view there is provided, in accordance with the invention, a measuring arrangement for measurement of optical properties of a sample to be investigated, as well as of analytes contained in the sample, in imaging techniques wherein the contrast agent comprises nano- or microparticles having anisotropic geometry and that the nano- or microparticles show geometric uniformity.

In an embodiment of the invention the at least one electromagnetic device is a pair of electromagnetic coils located equidistantly to the sample.

The following detailed description of preferred embodiments of the invention is merely exemplary in nature and is not intended to limit the invention or the application and uses of the invention. The invention will be described with reference to the following figures which are provided by way of explanation only.
Fig. 1 shows a schematic measuring arrangement;
Fig. 2 shows a measuring arrangement with two symbolic outcomes.

An ensemble of nano- or microparticles are used as contrast agents for imaging or in sensor technology. The nano- or microparticles have a minimum equivalent sphere diameter of about 10 nm and a maximum of about 100 µm. They also show a well-defined shape that differs from a sphere with a minimum aspect ratio of 1.3. Standard deviations of the geometry parameters are less than 50% of the mean values. The particles have a high geometric uniformity (the measurement of the optical absorption/scattering of the particles in solution shows characteristic peaks). Potential control of the spatial orientation of the particles is achievable so that the evaluation of the alignment ratio of an ensemble of these particles reveals a minimum value of 30%.

Hence, the nano- or microparticles have a well-defined shape that differs from a sphere and are designed in such a way that their spatial orientation can be controlled. The particles have a high geometric uniformity to allow measurements with a large number of particles in parallel (ensemble-based measurement). Excitation with polarized light can be used to generate a signal that depends on the orientation of the particles in relation to the direction of polarization of the excitation light. This means that at least two signals (or images) can be generated. In one signal there is a minimum of the signal of the contrast agent and in the other a maximum of the signal of the contrast agent. The different signal intensities result from different spatial orientations of the contrast agent in relation to the polarization direction of the exciting light. However, both signals have background noise. By subtracting one signal from the other, the background can then be eliminated, and a signal is obtained that is free from background.

In the state of art this works only for imaging modalities with high optical resolution and/or low concentration of the contrast agent. _{[SS3]}In the case of the method presented here, all nano- or microparticles have the same shape and can therefore be viewed as an ensemble, which allows for a higher particle concentration and, thus, for ensemble-based measurements. This is the only way to obtain higher signal intensities and signal-to-noise ratios. Within the resolution limit of the chosen detection scheme (either optical or acoustical) a larger number of particles can be used. The proposed method therefore provides a significant signal-to-noise improvement for light scattering-based detection, OCT, photoacoustic imaging, Raman spectroscopy.

Essential features of the invention are, thus, that a measurement method for the reduction or suppression of unwanted background is provided based on non-spherical nano- or microparticles employing an optical interrogation that exploits the orientation-dependent optical scattering (elastic or inelastic) and/or absorption characteristics of the nano- or microparticles with respect to polarized light. The non-spherical nano- or microparticles do have a well-defined shape with high geometric uniformity, which is a prerequisite for ensemble-based measurements.

### Advantages of additional optional features:

- Nano- or microparticles that show plasmon resonances:
   Plasmon resonances strongly increase absorption and scattering characteristics of micro- and nanoparticles. In case of Raman spectroscopy, the plasmons provide a field enhancement close to the particle surface, which strongly increases the Raman scattering efficiency.
- Nano- or microparticles with magnetic properties:
   Magnetic properties of nano- or microparticles allows the manipulation of their orientation in an external magnetic field.
- Excitation light that is polarized:
   o Control of the polarization of the excitation light:
      Instead of changing the orientation of the particles, it is also possible to change the orientation of the polarization axis. This avoids the implementation of rotatable external magnetic or electric fields and therefore can simplify the measurement setup.
   ∘ Alternatively, non-polarized light can be used for excitation in combination with a polarization filter 3 after the sample 5 (in the case of OCT or other light scattering-based detection schemes):
      In the case that a non-polarized light source is used, this configuration offers an alternative to positioning the polarization filter 3 in front of the sample 5. This is only possible if the resulting optical output signal is correlated to the particle orientation.
- Signal detection with simultaneous knowledge of the current orientation of the particles:
   The local dependence of the hydrodynamic resistance of the particles with respect to their surrounding can be exploited for extracting additional information out of the measurement signal such as viscosity.

The method is based on nano- or microparticles which have a non-spherical shape (anisotropic geometry). In addition, the method is based on the use of polarized light.

The anisotropic geometry of the particles and the use of polarized light make it possible to obtain scattering and/or absorption of the particles depending on their relative spatial orientation with respect to the polarization direction.

The alignment of the particles takes place via an external magnetic or electric field. The external field requires to be homogenous, i.e. with a low-gradient to avoid movement of the particles and to allow for a larger field of view. A homogenous low-gradient magnetic field can be generated by two permanent magnets located equidistant to the sample or by a pair of coils 10A, 10B (preferably arranged in the Helmholtz geometry). A homogenous low-gradient electric field can be generated by two electrodes located equidistant to the sample 5.

Polarized light is used to achieve an optical interaction with the particles that depends on their orientation. The high geometric uniformity of the particles ensures that all particles in the ensemble have the same orientation with respect to the externally applied magnetic or electric field. The optical measurement beam 2 can therefore excite many particles in parallel resulting in an increased signal strength without smearing out the orientation dependent effect.

The scattering and absorption strength of the particles depends on the alignment of the main axis to the polarization direction - parallel alignment of the main axis to the polarization provides a maximum of the optical effect and perpendicular alignment a minimum. In both cases, however, there is always background noise that is independent of the particles used. If one now measures the optical effects in both orientations, one can subtract them from one another and thus eliminate all noise and all background effects. On the one hand, this can be done statically by recording a signal in the case of a particle alignment, then rotating the alignment of the particles and recording a signal again. On the other hand, this can also be done dynamically by modulating the particle orientation and recording a current signal. A rotating magnetic or electric field can be used to generate a rotation of the particles, which leads to a measurement signal that is modulated with the excitation frequency (or a multiple thereof). One can then extract exactly the signal from the total signal that corresponds to the excitation frequency, which again filters out noise.

### Specific application examples

Cylindrical rod-shaped nano- or microparticles can be employed as contrast agent. The particles are ferromagnetic with their magnetic moment oriented along the long cylinder axis. The nanoparticles are dispersed in solution or a biological medium. If an external homogenous magnetic field is switched on, the nano- or microparticles' magnetic moment and therefore, the long axis of the particles, will be oriented parallel to the external magnetic field. The orientation of the particles depends on the direction of the external magnetic field.

For the optical measurement, linearly polarized light is used for optical excitation. The scattering and absorption strength of the particles depends on the alignment of the main nano- or microparticles cylinder axis to the polarization direction - parallel alignment of the main axis to the polarization provides a maximum of the optical effect and perpendicular alignment a minimum.

### Example 1:

If photoacoustic microscopy is carried out, the intensity of the photoacoustic signal depends on the optical absorption of the nano- or microparticle contrast agent. This means that a maximum of the photoacoustic signal is observed for particles aligned parallel to the direction of polarization and vice versa. Here, the excitation light of the photoacoustic measurement setup can be provided by a laser of 5 mW output power with a wavelength of 532 nm and is linearly polarized with a polarization extinction ratio of 20 dB. The beam diameter at the focal plane is 2µm. The nanoparticles are located in the region of interest in a specific sample to be able to act as contrast agent. The sample 5 can be a tissue section composed of various cell types, where the nanoparticles are fixed to a chosen cell type. Nanoparticles can be of cylindrical shape with a length of 80 nm and a diameter of 6 nm and can be composed of cobalt to show ferromagnetic properties. The standard deviation of both, the length and the diameter can be 10% of the mean value. The particle possesses a shell of gold with a mean thickness of 5 nm and 10% standard deviation. Standard detection of the photoacoustic pulse signal can be employed by using a piezoelectric transducer. The particle contrast agent can be oriented by an external magnetic field supplied by two orthogonal pairs of Helmholtz coils, which provide magnetic field strengths of 10 mT. The two coil pairs provide an orientation of the magnetic fields parallel and perpendicular to the direction of polarization. If a microscope image is now obtained for each of the two orientations of the nanoparticles, an image with a maximum signal of the particles and an image with a minimum signal are obtained. The acquisition time of a single image can be one minute. Both images have the same image background, which stems from the cells that are not labeled by nano- or microparticle contrast agent. The background can then be removed by subtracting the image with the minimum signal from the image with the maximum signal. The final background-free image therefore only has features where there are nano- or microparticles. This means that the location of the cells of interest are highlighted while the background is free from signal. Another example is the dispersion of the contrast agent within the blood stream of an animal model to highlight the blood vessels. Here, the resulting image will show the blood vessels and will not be obscured by the surrounding tissue. The same experimental setting accounts for optical coherence tomography. Here, the difference is that the measurement signal is an optical one depending on the scattering behavior of the nanoparticles.

### Example 2:

The spatial distribution of the concentration of a specific analyte molecule in a solution or in tissue can be measured by optical coherence tomography (OCT) and alternating external magnetic fields. The sample 5 can be a liquid (e.g. buffer solution or complex blood sample) containing the analyte molecule. The latter can be protein. Nanoparticles can be of cylindrical shape with a length of 80 nm and a diameter of 6 nm and can be composed of cobalt to show ferromagnetic properties. The standard deviation of both, the length and the diameter can be 10% of the mean value. The nanoparticle possesses a shell of gold with a mean thickness of 5 nm and 10% standard deviation. The nanoparticles need to be functionalized with an antibody that is selectively binding the protein of interest. Here, the external magnetic field is rotating at a frequency of 1000 Hz. The external rotating magnetic field is supplied by two orthogonal pairs of Helmholtz coils, which provide magnetic field strengths of 10 mT and are fed by sinusoidal input currents that are phase shifted by 90° to generate a rotating magnetic field. A prerequisite for this mode is that the sampling rate of the OCT is much higher than the rotational frequency of the magnetic field (e.g. 100x higher). Here, the excitation light of the OCT measurement setup can be provided by a laser of 5 mW output power with a wavelength of 1300 nm and is linearly polarized with a polarization extinction ratio of 20 dB. The beam diameter at the focal plane is 2µm. A standard OCT measurement setup can be employed including a reference arm and a dual balanced photodetector. The rotation of the external magnetic field induces a rotation of the nanoparticles and, thus, a modulation of the scattering signal. The modulation frequency of the optical scattering signal in case of cylindrical nanoparticles is two times the frequency of the external magnetic field (parallel alignment of the nanoparticles every 180 ° of the external rotating magnetic field and, thus, a doubling of the frequency of the external magnetic field). Thus, the frequency of the detected sinusoidal OCT signal is 2000 Hz. By comparing the orientation of the external magnetic field and the actual nanoparticle orientation measured by OCT, one can calculate the phase lag between the nanoparticle and the external magnetic field. The voltage via a shunt resistor in the electronic circuit feeding the coils with input current can be employed as reference to determine the momentary orientation of the external magnetic field. A lock-in amplifier technique can be employed to measure the phase lag by feeding the optical signal into the sample input and by providing the shunt resistor voltage to reference input. The Lock-in amplifier needs to work at its 2F mode (i.e. doubling the frequency of the reference signal). This phase lag depends on the drag of the nanoparticles and thus, on their volume. Binding of the analyte protein results in a change of the nanoparticle volume and its drag, which itself results in an increased phase lag between the external magnetic field and the nanoparticles. Measurements of the frequency-modulated OCT signal allow to determine the phase lag, which indicates the presence of the analyte molecule. From the phase lag, the concentration of the analyte molecule can be derived (the higher the number of analyte molecules bound to the nanoparticle surface the higher the increase of the phase lag). In addition, the tomography measurement modality allows to deduce the location and allows to conduct a concentration mapping of the analyte. The overall measurement principle also works for small analyte molecules in a competitive assay format where the nanoparticles are pre-loaded with a bigger molecule (e.g. a spherical nanoparticle or a high-molecular-weight polymer). Here, the analyte molecules replace the bigger pre-bound species. This results in the decrease of the observed phase lag. The presence and the concentration, including a concentration mapping, of the analyte molecule is only accessible due to the proposed measurement mode.

### Example 3:

The measurement of a specific analyte molecule in solution and a reduction of background signal can also be targeted by surface-enhanced Raman spectroscopy (SERS). Here, the nano- or microparticles act as signal enhancing factor for the Raman scattering signal. To that end, the plasmonic particle properties are exploited. Enhancement of the Raman signal is achieved due to the local electric field enhancement in close vicinity of the nanoparticle surface. The sample 5 can be a liquid sample (e.g. water) containing the analyte molecule. The latter can be a small molecule showing Raman activity (e.g. polychlorinated biphenyls). nano- or microparticles can be of cylindrical shape with a length of 80 nm and a diameter of 6 nm and can be composed of cobalt to show ferromagnetic properties. The standard deviation of both, the length and the diameter can be 10% of the mean value. The particle possesses a shell of gold with a mean thickness of 5 nm and 10% standard deviation. The nano- or microparticles need to be functionalized with a porous shell (e.g. molecularly imprinted polymer) that allows the analyte molecule to be trapped near the particle surface. Here, the excitation light of the measurement setup can be provided by a laser of 5 mW output power with a wavelength of 785 nm and is linearly polarized with a polarization extinction ratio of 20 dB. The beam diameter at the focal plane is 2µm. A standard Raman measurement setup can be employed including a diffraction grating and a CMOS camera as spectrometer and an optical filter in front to block the excitation wavelength. Cylindrical particles exhibit two plasmon resonance modes (parallel and perpendicular to the main cylinder axis). Both plasmon resonance modes result in the formation of hot spots (spots close to the nanoparticle surface with high electric field enhancement). Thus, the orientation of the nanorods allows for exciting a specific plasmon resonance mode and therefore, the formation of distinct hot spots. Alignment of the cylindrical particle parallel to the polarization of the incoming light favors the formation of hot spots on the tips of the cylinder, while a parallel orientation favors the formation of hot spots along the side surface of the cylinder. Raman-active molecules present in the hot spot regions generate characteristic Raman scattering spectral fingerprints. The particle surface coating allows to bind a target molecule close to the particle surface so that the analyte molecule is concentrated in the region of the plasmonic hot spot. The signal intensity of the Raman signal depends on the orientation of the nano- or microparticle. This can be exploited in analogy of Example 1 and Example 2 either by static external fields and the subtraction of the background, or by using rotating external fields and by inducing a frequency modulation of the Raman signal. For the latter, the resulting Raman signal for each detected wavelength can be frequency filtered (e.g. Fourier analysis of the time dependent signal for each wavelength) to deduce the part of the signal that corresponds to the nano- or microparticle motion. This results in background-free visualization of the Raman spectrum of the chosen analyte and minimizes autofluorescence background.

### Example 4:

The above-mentioned measurement examples 1-3 can also be realized in a modified way by keeping the external magnetic field static in one direction and by changing the direction of polarization of the interrogating light beam instead of changing the orientation of the nano- or microparticles .

### Example 5:

The above-mentioned measurement examples 1-3 can also be realized in a modified way by keeping the external magnetic field static in one direction and by using unpolarized light. This measurement mode requires a polarization filter 3 in the light path after the sample 5 has been excited.

### Technical sketches:

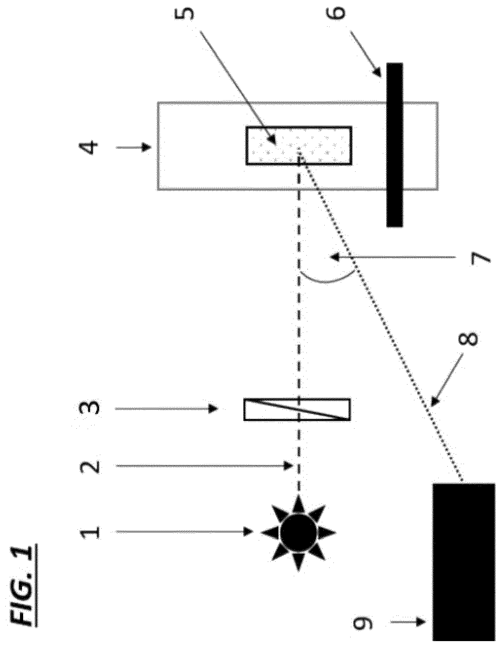

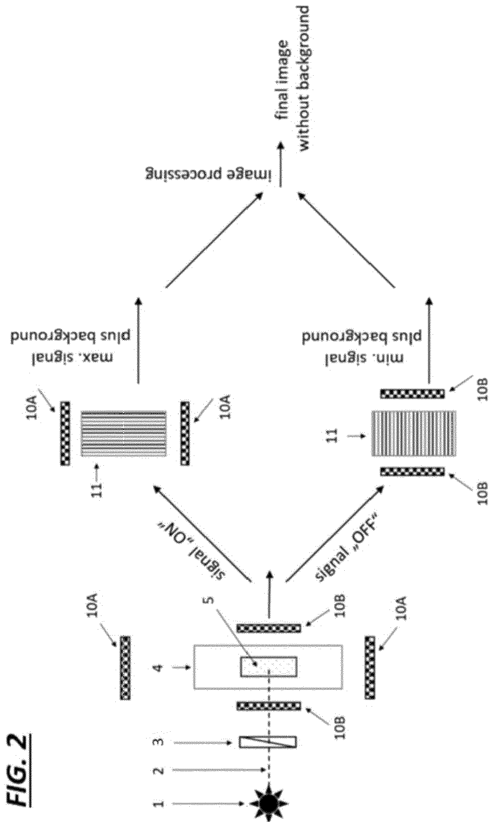

One possible measurement setup (see FIG. 1) consists of a light source 1 which generates a light beam 2. This light beam, if it does not already have a direction of polarization (for example by using a laser as the light source), then passes through a polarization filter 3. The light beam is directed onto the measuring cell 4 and hits the sample 5 there. This sample 5 contains the particulate contrast agent, which can move freely at least around its own axis. The spatial orientation of the contrast agent can be controlled by external excitation by means of a suitable electromagnetic device 6. The optical absorption and / or scattering of the contrast agent results in a signal (optical or also acoustical), which strikes a suitable detector 9 at an angle 7 and after a signal path 8 of any length. A possible measurement method, as it is also described above as an application example (Example 1), is shown schematically in FIG. 2. The electromagnetic device for manipulating the spatial orientation of the contrast medium is in this case a pair of electromagnetic coils (pair 10A and pair 10B) and the contrast agent consists of elongated magnetic nano- or microparticles with a spatial orientation 11. The magnet causes the nano- or microparticles to rotate and aligns them either parallel to the direction of polarization (signal "ON" path in Fig. 2) or at perpendicular angles to the direction of polarization (signal "OFF" path in Fig. 2). If an optical or acoustic detection is now carried out and an image is recorded, a maximum measurement signal is obtained on one hand and a minimum measurement signal on the other hand. Both also contain the background noise. Subsequent image processing subtracts the "OFF" signal from the "ON" signal and thus removes the background. The final image has no background noise. In the case of a continuously rotating external magnetic field, Fourier analysis of the time dependent measurement signal can be used to suppress the background components of the signal.

### References

[1] A. Krumholz, D.M. Shcherbakova, J. Xia, L.V. Wang, V.V. Verkhusha, Multicontrast photoacoustic in vivo imaging using near-infrared fluorescent proteins, Sci. Rep. 4 (2014) 3939. https://doi.org/10.1038/srep03939.
[2] J.M. Tucker-Schwartz, T.A. Meyer, C.A. Patil, C.L. Duvall, M.C. Skala, In vivo photothermal optical coherence tomography of gold nanorod contrast agents, Biomed. Opt. Express 3 (2012) 2881-2895. https://doi.org/10.1364/BOE.3.002881.
[3] P.-C. Li, C.-R.C. Wang, D.-B. Shieh, C.-W. Wei, C.-K. Liao, C. Poe, S. Jhan, A.-A. Ding, Y.-N. Wu, In vivo photoacoustic molecular imaging with simultaneous multiple selective targeting using antibody-conjugated gold nanorods, Opt. Express 16 (2008) 18605. https://doi.org/10.1364/OE.16.018605.
[4] S.A. Boppart, A. Wei, Multifunctional plasmon-resonant contrast agents for optical coherence tomography, Patent US7610074B2 (2004).
[5] A.L. Oldenburg, B.E. Applegate, J.A. Izatt, S.A. Boppart, Molecular OCT Contrast Enhancement and Imaging, in: E. Greenbaum, W. Drexler, J.G. Fujimoto (Eds.), Optical Coherence Tomography, Springer Berlin Heidelberg, Berlin, Heidelberg, 2008, pp. 713-756.
[6] A. Oldenburg, F. Toublan, K. Suslick, A. Wei, S. Boppart, Magnetomotive contrast for in vivo optical coherence tomography, Opt. Express 13 (2005) 6597-6614.
[7] A.L. Oldenburg, V. Crecea, S.A. Rinne, S.A. Boppart, Phase-resolved magnetomotive OCT for imaging nanomolar concentrations of magnetic nanoparticles in tissues, Opt. Express 16 (2008) 11525-11539.
[8] Y. Jin, C. Jia, S.-W. Huang, M. O'Donnell, X. Gao, Multifunctional nanoparticles as coupled contrast agents, Nat. Commun. 1 (2010) 41. https://doi.org/10.1038/ncomms1042.
[9] J. Li, B. Arnal, C.-W. Wei, J. Shang, T.-M. Nguyen, M. O'Donnell, X. Gao, Magneto-optical nanoparticles for cyclic magnetomotive photoacoustic imaging, ACS Nano 9 (2015) 1964-1976. https://doi.org/10.1021/nn5069258.
[10] M. O'Donnell, X. Gao, Method and system for background suppression in magneto-motive photoacoustic imaging of magnetic contrast agents, Patent US8701471B2 (2011).

### List of Reference Signs

- 1: light source
- 2: light beam
- 3: polarizer
- 4: measuring cell
- 5: sample
- 6: electromagnetic device
- 7: angle
- 8: signal path
- 9: detector
- 10A: electromagnetic coil
- 10B: electromagnetic coil
- 11: orientation of particles

## Claims

1. A measurement method for the reduction or suppression of unwanted background in imaging techniques based on a contrast agent employing an optical interrogation that exploits the orientation-dependent optical scattering and/or absorption characteristics of the contrast agent with respect to light, which method comprises the steps of:
- providing a sample (5) to be analyzed in an optical path between a light source (1) and a detector (9),
- providing as contrast agent nano- or microparticles with magnetic and/or electrostatic properties within the sample,
- generating a magnetic and/or electrostatic field of at least one spatial direction,
- generating at least two signals or images, wherein in one signal there is a minimum of the signal of the contrast agent and in the other a maximum of the signal of the contrast agent exploiting the orientation dependent polarization characteristics of the contrast agent,
- subtracting one signal from the at least one other signal to eliminate the background,
**characterised in that**
the nano- or microparticles used as contrast agent have anisotropic geometry and that the nano- or microparticles show geometric uniformity.

2. Method according to claim 1, wherein cylindrical rod-shaped nano- or microparticles are used.

3. Method according to claim 1 or 2, wherein the excitation of the sample (5) and the nano- or microparticles is conducted with polarized light.

4. Method according to claim 3, wherein the polarization is controlled in a predefined way.

5. Method according to claim 1 or 2, wherein non-polarized light is used for excitation in combination with a polarization filter (3) after the sample (5).

6. Method according to one of claims 1 to 5, wherein the optical scattering is at least partly inelastic.

7. Method according to one of claims 1 to 6, wherein nano- or microparticles show plasmon resonances.

8. Method according to one of claims 1 to 7, wherein an external homogenous low-gradient magnetic field is generated by two permanent magnets located equidistant to the sample (5) or by a pair of coils (10A, 10B), preferably arranged in a Helmholtz geometry.

9. Method according to claim 8, wherein the magnetic field is a time-modulated field.

10. Method according to one of claims 1 to 7, wherein an external homogenous low-gradient electric field is generated by two electrodes located equidistant to the sample (5).

11. Method according to claim 10, wherein the electric field is a time-modulated field.

12. Method according to claims 1 to 11, wherein the signal detection is conducted with simultaneous knowledge of the current orientation of the nano- or microparticles.

13. Method according to claims 1 to 12, wherein the detection method is light scattering-based detection schemes.

14. Method according to claims 1 to 12, wherein the detection method is light absorption-based detection schemes.

15. Method according to claims 1 to 12, wherein the detection method is optical coherence tomography.

16. Method according to claims 1 to 12, wherein the detection method is photoacoustic imaging.

17. Method according to claims 1 to 12, wherein the detection method is surface enhanced Raman spectroscopy.

18. Measuring arrangement for measurement of optical properties of a sample to be investigated, as well as of analytes contained in the sample, in imaging techniques comprising a measuring cell (4) containing the sample (5), wherein the measuring cell (4) is placed in an optical path between a light source (1) and a detector (9) for the detection of scattered light coming from the light source (1) and interacted with the sample (5), wherein sample (5) contains a contrast agent, which contrast agent comprises particles that are able to be aligned spatially depending on the presence of a predetermined magnetic or electric field, and wherein at least one electromagnetic device (6, 10A, 10B) is located adjacent to the measuring cell (4) to be able to act on the contrast agent, **characterized in that** the contrast agent comprises nano- or microparticles having anisotropic geometry and that the nano- or microparticles show geometric uniformity.

19. Measuring arrangement according to claim 18, **characterized in that** the electromagnetic device (6, 10A, 10B) is a pair of electromagnetic coils (10A, 10B) located equidistantly to the sample (5).
